# EUROPÄISCHE PATENTSCHRIFT

(11) **EP 1 421 917 B1**
(45) Veröffentlichungstag und Bekanntmachung des Hinweises auf die Patenterteilung: **26.03.2008**
(21) Anmeldenummer: 03025246.4
(22) Anmeldetag: 05.11.2003
(51) Int. Cl.: A61F 2/16

(54) **Vorrichtung zum Halten einer faltbaren Intraokularlinse**
Device to hold a foldable intraocular lens
Dispositif de maintien d'une lentille intraoculaire

(30) Priorität: 25.11.2002 DE 10254944
(43) Veröffentlichungstag der Anmeldung: 26.05.2004
(73) Patentinhaber: *Acri.Tec AG Gesellschaft für ophthalmologische Produkte, 16761 Hennigsdorf (DE)
(72) Erfinder: Serester, Alexander, Dipl.-Ing., 93077 Bad Abbach (DE); Nemitz, Bernd, Dipl.-Ing., 16567 Schönfliess (DE)
(74) Vertreter: Nöth, Heinz

(56) Entgegenhaltungen:
- EP-A- 1 042 999
- EP-A- 1 114 623
- US-A- 5 454 818

## Beschreibung

### [Beschreibung]

Die Erfindung betrifft eine Vorrichtung zum Halten einer faltbaren Intraokularlinse nach dem Oberbegriff des Patentanspruches 1.

### [Stand der Technik]

Eine derartige Vorrichtung ist aus der EP 1 042 999 A1 bekannt. Die bekannte Vorrichtung besitzt zwei zueinander schwenkbare Haltearme, zwischen denen eine Vorspannung wirkt, mit denen die beiden Arme auseinandergespreizt werden. An ihren freien Enden besitzen die Haltearme Stütz- und Befestigungsmittel, mit denen die Intraokularlinse im ungefalteten Zustand, beispielsweise in einem Behälter angeordnet gehalten wird. Beim Entfernen der Haltearme aus dem Behälter wird durch zusätzliche Betätigungs- und Verriegelungsmittel auf die Haltearme eine Belastung so ausgeübt, dass sie aufeinander zu bewegt werden, wobei die Linse gefaltet wird. Zum Falten der Intraokularlinse sind somit bei der bekannten Vorrichtung zusätzliche Mittel erforderlich, mit denen die Haltearme aufeinander zu geschwenkt werden, um das Falten der Linse zu bewirken.

### [Aufgabe der Erfindung]

Aufgabe der Erfindung ist es, eine Vorrichtung der eingangs genannten Art zu schaffen, bei welcher mit verringertem Aufwand die Linse im ungefalteten Zustand im Behälter aufgewahrt wird und mit welcher ein vereinfachtes Falten der Linse erzielt wird.

Diese Aufgabe wird erfindungsgemäß durch die kennzeichnenden Merkmale des Patentanspruches 1 gelöst.

Die Unteransprüche beinhalten vorteilhafte Weiterbildungen der Erfindung.

Bei der Erfindung werden die freien Enden der Haltearme durch die Vorspannung in Richtung aufeinander zu vorgespannt. Die Vorspannung ist so bemessen, dass beim Entfernen der Haltearme aus dem Behälter, die zwischen den Haltearmen gefaltete Linse selbsttätig durch die Vorspannkraft gefaltet wird. Im Behälter ist eine Spreizeinrichtung vorgesehen, durch welche die Haltearme gegen die Vorspannung zum Halten der Linse im ungefalteten Zustand auseinandergespreizt werden.

Vorzugsweise sind die beiden Haltearme aus einem Stück gebildet. Die Haltearme können in einer Verbindungsstelle schwenkbar verbunden sein, wobei die Spreizeinrichtung an wenigstens einem der beiden Haltearme angreift. Die Vorspannkraft kann in der Verbindungsstelle gebildet werden, wobei die Verbindungsstelle durch einen Steg, mit dem die beiden Haltearme miteinander verbunden sind, gebildet sein kann. In der Verbindungsstelle kann eine Schwachstelle vorgesehen sein. Durch die Verbindungsstelle werden die beiden Haltearme nach Art einer Zange miteinander verbunden.

Die Vorspannkraft kann in bevorzugter Weise auch durch ein federelastisches Element gebildet werden. Das federelastische Element drückt die beiden Enden, zwischen denen die Linse gehalten wird, aufeinander zu. Das federelastische Element kann als Kugel aus einem federelastischen Material beispielsweise Elastomer oder aus Silikonkautschuk hergestellt sein. Es kann als Blattfeder oder mechanische Schraubenfeder und dergleichen ausgebildet sein.

Wenigstens einer der beiden Haltearme kann als einseitiger oder zweiseitiger Hebel ausgebildet sein. An diesem Hebel greift die Spreizeinrichtung an.

Dieser wenigstens eine Haltearm kann als geradliniger zweiseitiger Hebel ausgebildet sein. Es ist jedoch auch möglich, den wenigstens einen Haltearm, auf welchen die Spreizeinrichtung wirkt, als einseitigen oder zweiseitigen Winkelhebel auszubilden, wobei die Spreizeinrichtung auf den abgewinkelten Hebelarm zur Einwirkung kommt.

Die Spreizeinrichtung kann in einfacher Weise als ortsfester Anschlag im Gehäuseinnern vorgesehen sein. Hierzu kann die Spreizeinrichtung als in das Gehäuseinnere radial und/oder axial ragender Vorsprung ausgebildet sein. Hierbei kann es sich um einen im Gehäuseinnern umlaufenden Vorsprung handeln. Es ist jedoch auch möglich, diesen Vorsprung am Innenumfang des Gehäuses nur an der Stelle vorzusehen, an welcher er zum Spreizen der Haltearme auf wenigstens einen der Haltearme einwirkt.

Der andere Hebelarm ist vorzugsweise mit einem Verschlusselement, beispielsweise einem Schraubverschluss des Behälters verbunden. Es ist jedoch auch möglich, beide Haltearme an einem gemeinsamen stabförmigen Halteelement oder einem Stumpf, der ebenfalls mit dem Behälterverschluss verbunden ist, anzuordnen, wobei das Halteelement bzw. der Stumpf aus einem Stück mit den beiden Haltearmen gebildet sein kann.

Ferner kann an einem Haltearm ein den Faltungsvorgang der Linse einleitender Impulsgeber vorgesehen sein. Dieser Impulsgeber kann beispielsweise als Vorsprung, als Höcker oder dergleichen ausgebildet sein. Es können auch an beiden Haltearmen derartige Impulsgeber vorgesehen sein. Beim Entfernen der Haltearme aus dem Behälter wirkt dieser Impulsgeber auf die in der Halteeinrichtung gehaltene Intraokularlinse so ein, dass diese in der gewünschten Richtung gefaltet wird. Die gefaltete Linse kann dann mit Hilfe eines Implantationswerkzeugs, beispielsweise einer Pinzette für die Implantation aus der Halteeinrichtung entnommen werden.

### [Beispiele]

Anhand der Figuren wird an einem Ausführungsbeispiel die Erfindung noch näher erläutert.

Es zeigt
- Fig. 1: in schematischer Darstellung ein erstes Ausführungsbeispiel mit in einem Behälter angeordneter Halteeinrichtung für die Intraokularlinse;
- Fig. 2: in schematischer Darstellung das Ausführungsbeispiel der Figur 1 mit aus dem Behälter entfernter Halteeinrichtung und gefalteter Intraokularlinse;
- Fig. 3: ein zweites Ausführungsbeispiel mit im Behälter angeordneter Halteeinrichtung; und
- Fig. 4: ein drittes Ausführungsbeispiel mit im Behälter angeordneter Halteeinrichtung.

In den Figuren sind Haltevorrichtungen zum Halten einer faltbaren Intraokularlinse 6 dargestellt. Die dargestellten Ausführungsbeispiele besitzen zwei Haltearme, nämlich einen Haltearm 1 und einen Haltearm 2. An freien Armenden 3 und 4 sind Halteeinrichtungen 7 und 8 ausgebildet, zwischen denen die Intraokularlinse gehalten wird. Die Halteeinrichtungen 7 und 8 sind als V-förmige Vertiefungen an den aufeinander zu gekehrten Innenseiten der Haltearme 1 und 2 im Bereich der freien Armenden 3 und 4 vorgesehen.

Bei den beiden Ausführungsbeispielen sind die beiden Haltearme 1 und 2 einstückig ausgebildet und über eine Verbindungsstelle 9 miteinander verbunden.

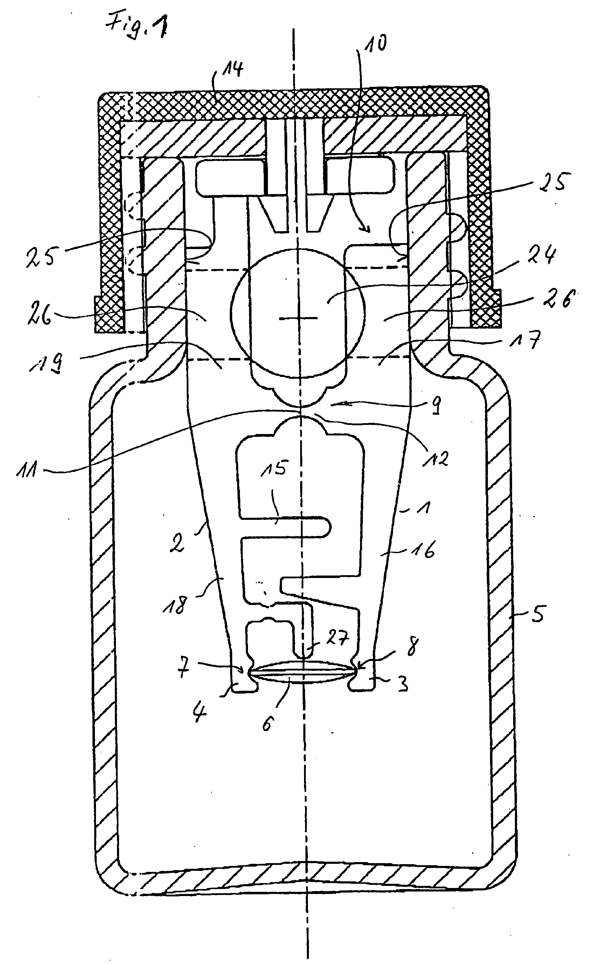

Bei den Ausführungsbeispielen der Fig. 1 bis 3 sind die beiden Haltearme, welche als zweiseitige Hebel ausgebildet sind, über einen Steg 12, welcher die Verbindungsstelle 9 darstellt, miteinander verbunden.

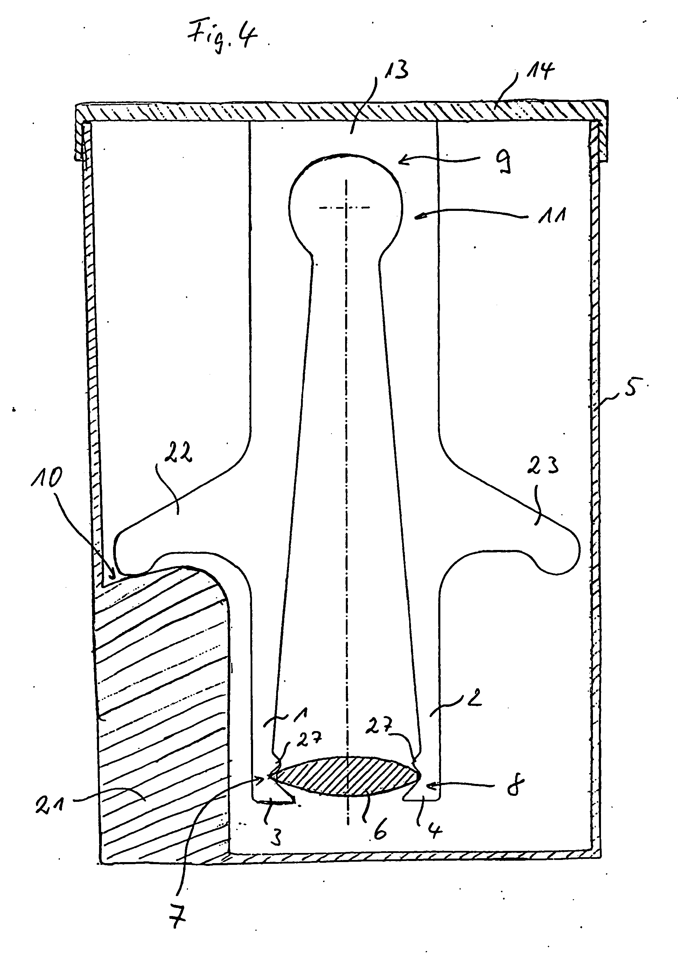

Beim Ausführungsbeispiel der Fig. 4 laufen die beiden Haltearme 1 und 2 in einem Verbindungsstumpf 13 zusammen. Im Bereich der Verbindungsstelle 9 besitzen die Ausführungsbeispiele eine Schwachstelle 11. Durch die Schwachstelle 11 ist die Schwenkbarkeit der Haltearme im Bereich der jeweiligen Verbindungsstelle 9 erleichtert.

Bei den Ausführungsbeispielen der Fig. 1 bis 3 sind die beiden Haltearme als zweiseitige Hebel ausgebildet, wobei der Haltearm 1 aus einem ersten Hebelarm 16 und einem zweiten Hebelarm 17 besteht. Der Haltearm 2 besteht aus einem ersten Hebelarm 18 und einem zweiten Hebelarm 19. Die Halteeinrichtungen 7 und 8 befinden sich an freien Enden 3, 4 der ersten Hebelarmen 16 und 18. Die Haltearme 1 und 2 sind ähnlich wie bei einer Zange miteinander verbunden.

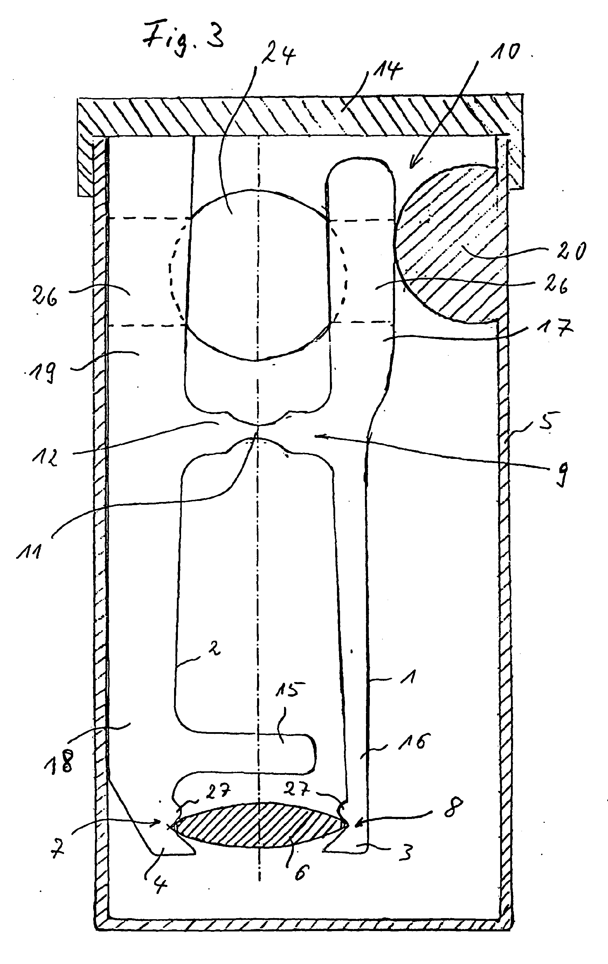

Beim Anordnen der Haltevorrichtungen in einem Behälter 5 werden die ersten Hebelarme 16 und 18 durch eine Spreizeinrichtung, welche die zweiten Hebelarme 17, 19 zusammendrückt, auseinandergespreizt. Die in der Fig. 3 dargestellte Spreizeinrichtung beinhaltet einen radialen Vorsprung 20, welcher als umlaufender Vorsprung an der Innenseite der Behälterwand oder als örtlich begrenzter Vorsprung 20 ausgebildet sein kann. Im Querschnitt besitzt der radiale Vorsprung 20 ein etwa halbkugelförmiges Profil. An der Außenfläche bildet der Vorsprung 20 eine Anlagefläche, an welcher der zweite Hebelarm 17 des Haltearms 1 anliegt. Durch den radialen Vorsprung 20 wird der zweite Hebelarm 17 nach innen gedrückt, wodurch der erste Hebelarm 16 um die Verbindungsstelle 9 im Bereich des Steges 12 nach außen geschwenkt wird. Bei dem in der Figur 1 dargestellten Ausführungsbeispiel liegen die zweiten Hebelarme 17 und 19 der Haltearme 1 und 2 an der umlaufenden Innenwand des Behälters im Bereich von Anschlagflächen 25 an. Die Anschlagflächen 25 können auf einer umlaufenden Verengung des Behälters, beispielsweise einem Flaschenhals vorgesehen sein. Auch hierdurch werden die zweiten Hebelarme 17, 19 aufeinander zu und die ersten Hebelarme 16 und 18 nach außen geschwenkt bzw. gespreizt. Damit ist gewährleistet, dass die Intraokularlinse 6 zwischen den Halteeinrichtungen 7 und 8 im ungefalteten Zustand im Bereich der freien Enden 3 und 4 der Haltearme 1 und 2 gehalten wird, wie es in den Fig. 1 und 3 dargestellt ist.

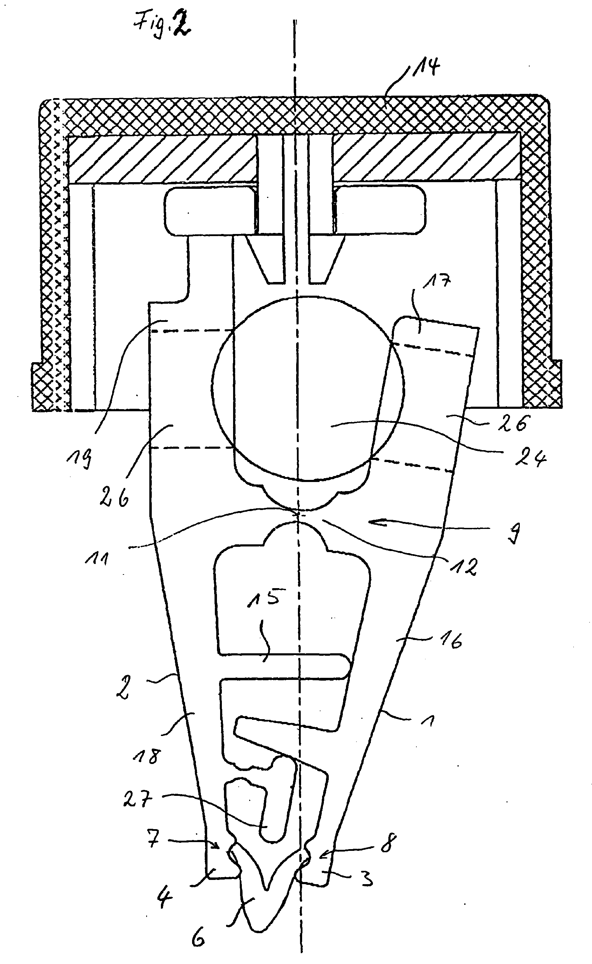

Bei den Ausführungsbeispielen der Fig. 1 bis 3 ist der eine Haltearm 1 gegenüber einem Behälterverschluss 14 beweglich angeordnet. Der andere Haltearm 2 ist mit dem Behälterverschluss 14, welcher beispielsweise auf den Behälter 5 aufgeschraubt oder aufgeclipst werden kann, fest verbunden. Beim Einsetzen der Haltevorrichtung in das Behälterinnere wird, wie oben erläutert, der erste Hebelarm 16 vom ersten Hebelarm 18 weggespreizt, da der radiale Vorsprung 20 bzw. der Behälterverengung als Spreizeinrichtung auf den zweiten Hebelarm 17 wirkt. Beim Entfernen der Haltevorrichtung aus dem Behälter 5 kommt der radiale Vorsprung 20 bzw. die verengte Behälterwand an den Anschlagflächen 25 außer Eingriff mit dem zweiten Hebelarm 17, wodurch die im Bereich der Verbindungsstelle 9 wirkende Vorspannkraft zur Auswirkung kommt. Diese Vorspannkraft drückt die beiden ersten Hebelarme 16 und 18 aufeinander zu, wie es in der Figur 2 dargestellt ist. Die Aufeinanderzubewegung der beiden Hebelarme 16 und 18 kann durch einen Abstandhalter 15, der zwischen diesen beiden Hebelarmen wirkt, begrenzt werden. Auf diese Weise erreicht man beim Entfernen der Haltevorrichtung aus dem Behälter 5 eine selbsttätige Faltung der Intraokularlinse 6 aufgrund der Vorspannung, die im Material der zangenartig miteinander verbundenen Haltearme 1 und 2 wirkt und/oder durch ein federelastisches Element 24 erzeugt wird.

Das federelastische Element 24 kann als Kugel aus einem federelastischen Material, beispielsweise einem Elastomer oder Silikonkautschuk oder dergleichen bestehen. Anstelle der Kugelform kann auch eine andere geeignete Form, z. B. Quader, Ellipsoid oder dergleichen, zum Einsatz kommen. Ferner kann als federelastisches Element eine Blattfeder, welche separat oder einstückig mit wenigstens einem der beiden Haltearme zwischen den beiden Hebelarmen 17 und 19 wirkt, ausgebildet sein. Ferner kann eine Schraubenfeder oder auch ein anderes mechanisches Federelement zur Erzeugung der Vorspannkraft verwendet werden. Bei den dargestellten Ausführungsbeispielen der Figuren 1 bis 3 ist das kugelförmige federelastische Element 24 in Ausnehmungen 26 der beiden zweiten Hebelarme 17 und 19 gehalten. Durch das federelastische Element 24 werden die zweiten Hebelarme 17 und 19 auseinandergedrückt und die ersten Hebelarme 16 und 18 vorgespannt, wenn die Haltearme 1, 2 im Behälter 5 angeordnet sind.

Anstelle der Ausbildung der Haltearme 1 und 2 und insbesondere des Haltearmes 1 als geradliniger zweiseitiger Hebelarm kann insbesondere der Haltearm 1 auch als zweiseitiger Winkelhebelarm ausgebildet sein, wobei vorzugsweise der zweite Hebelarm 17 abgewinkelt ist.

Bei dem in der Fig. 4 dargestellten Ausführungsbeispiel sind die beiden Hebelarme 1 und 2 als einseitige Hebel ausgebildet, die Winkelhebelarme 22 und 23 aufweisen. Die Schwenkachse der Haltearme 1 und 2 liegt im Bereich der Schwachstelle 11 bei der Verbindungsstelle 9. Die Schwachstelle ist teilkreisförmig im Material der Verbindungsstelle 9, in welcher die einstückig miteinander verbundenen Haltearme 1 und 2 zusammenlaufen, gebildet. Über einen Stumpf 13 sind die beiden Haltearme gemeinsam an dem Behälterverschluss 14 befestigt. Bei der Ausführungsform der Fig. 4 wird beim Einsetzen der Haltevorrichtung der Winkelhebelarm 22 des Haltearms 1 mit einem sich in axialer Richtung erstreckenden Vorsprung 21 in Anlage gebracht. Hierdurch wird der Haltearm 1 um die Verbindungsstelle 9 vom Haltearm 2 gegen die Vorspannkraft wegbewegt. Wie bei den Ausführungsbeispielen der Fig. 1 bis 3 wirkt die Vorspannkraft so, dass die beiden Haltearme 1 und 2 aufeinander zu vorgespannt werden. In dieser gespreizten Stellung der Haltearme 1 und 2 wird die Intraokularlinse 6 im Behälter 5 im ungefalteten Zustand zwischen den Halteeinrichtungen 7 und 8 im Bereich der freien Armenden 3 und 4 gehalten.

Beim Entfernen der Haltevorrichtung aus dem Behälter 5 wird, wie bei den Ausführungsbeispielen der Fig. 1 bis 3, der Haltearm 1 aufgrund der Vorspannkraft auf den Haltearm 2 zu bewegt. Bei den Ausführungsbeispielen der Figuren 1 bis 3 werden die zweiten Hebelarme 17, 19 durch die Vorspannung, insbesondere das federelastische Element 24 auseinander bewegt und die ersten Hebelarme 16, 18 aufeinander zu bewegt. Dabei wird die Intraokularlinse 6, wie die Figur 2 zeigt, gefaltet. Auch beim Ausführungsbeispiel der Fig. 4 kann ein zusätzliches, die beiden Haltearme 1 und 2 umspannendes Federelement zur Bildung oder Unterstützung der Vorspannkraft, mit welcher die selbsttätige Faltung der Linse 6 beim Entfernen der Haltevorrichtung aus dem Behälter 5 bewirkt wird, vorgesehen sein.

Es können auch zwei Vorsprünge 21 vorgesehen sein, welche auf beide Winkelhebelarme 22 und 23 einwirken. Ferner kann auch ein umlaufender Vorsprung vorgesehen sein, mit welchem ebenfalls auf die beiden Winkelhebelarme 22 und 23 zur Spreizung der Haltearme 1 und 2 eingewirkt wird.

Ferner kann an einem der beiden Haltearme 1 und 2 oder an beiden Haltearmen ein mechanischer Impulsgeber 27 vorgesehen sein. Dieser Impulsgeber 27 bewirkt, dass die Intraokularlinse 6 in einer bestimmten Richtung gefaltet wird, wie es in der Figur 2 beispielsweise dargestellt ist. Der Impulsgeber 27 kann als Stößel wie bei der Ausführungsform der Figuren 1 und 2 an einem der beiden Haltearme vorgesehen sein. Beim Ausführungsbeispiel der Figuren 1 und 2 ist der Impulsgeber 27 am Haltearm 2 vorgesehen.

Bei den Ausführungsformen der Figuren 3 und 4 sind die Impulsgeber 27 in der unmittelbaren Nähe der Halteeinrichtungen 7 und 8 an den freien Enden der Haltearme 1 und 2 vorgesehen. Durch den Impulsgeber 27 bzw. die Impulsgeber 27 ist gewährleistet, dass die Intraokularlinse beim Aufeinanderzubewegen der freien Enden 3 und 4 der Haltearme die Linse in Richtung nach außen bezüglich der Halteeinrichtungen 7 und 8 gefaltet wird, wie es in Figur 2 dargestellt ist. Hierdurch kann die gefaltete Linse 6, beispielsweise mit Hilfe einer Pinzette oder einem anderen Implantationswerkzeug leicht entnommen werden. Der Impulsgeber 27 bzw. die Impulsgeber 27 sind bezüglich der zwischen den Halteeinrichtungen 7 und 8 gehaltenen Intraokularlinse 6 so angeordnet, dass beim Aufeinanderzubewegen der freien Enden 3 und 4 der Haltearme 1 und 2 der gewünschte Impuls bzw. die gewünschten Impulse auf die Linse 6 ausgeübt wird, bzw. werden und die Linse 6 nach außen gerichtet gefaltet wird.

### [Bezugszeichenliste]

- 1: Haltearm
- 2: Haltearm
- 3: freies Armende
- 4: freies Armende
- 5: Behälter
- 6: Intraokularlinse
- 7: Halteeinrichtung
- 8: Halteeinrichtung
- 9: Verbindungsstelle
- 10: Spreizeinrichtung
- 11: Schwachstelle
- 12: Verbindungssteg
- 13: Stumpf
- 14: Behälterverschluss
- 15: Abstandhalter
- 16: erster Hebelarm
- 17: zweiter Hebelarm
- 18: erster Hebelarm
- 19: zweiter Hebelarm
- 20: radialer Vorsprung
- 21: axialer Vorsprung
- 22: Winkelhebelarm
- 23: Winkelhebelarm
- 24: federelastisches Element (Silikonkugel)
- 25: Anschlagfläche
- 26: Ausnehmung

## Patentansprüche

1. Vorrichtung zum Halten einer faltbaren Intraokularlinse mit zwei zueinander schwenkbaren Haltearmen, zwischen denen eine Vorspannung wirkt und welche, angeordnet in einem Behälter, in der Nähe der freien Enden oder an den freien Enden die Linse in ungefaltetem Zustand halten, wobei beim Entfernen aus dem Behälter die Linse durch Aufeinanderzubewegen der Haltearme gefaltet wird,
**dadurch gekennzeichnet, dass** die freien Enden (3, 4) der Haltearme (1, 2), welche durch die Vorspannung in Richtung aufeinander zu vorgespannt sind, bei der Anordnung im Behälter (5) gegen die Vorspannung zum Halten der Linse (6) im ungefalteten Zustand gespreizt sind und die Vorspannkraft so bemessen ist, dass beim Entfernen der Haltearme aus dem Behälter (5), die zwischen den Haltearmen (1, 2) gehaltene Linse (6) gefaltet wird.

2. Vorrichtung nach Anspruch 1,
**dadurch gekennzeichnet, dass** im Behälter (5) eine Spreizeinrichtung (20; 21) vorgesehen ist, durch welche die Haltearme (1, 2) gegen die Vorspannung zum Halten der Linse im ungefalteten Zustand auseinander gespreizt sind.

3. Vorrichtung nach Anspruch 1 oder 2,
**dadurch gekennzeichnet, dass** die beiden Haltearme (1, 2) aus einem Stück gebildet sind.

4. Vorrichtung nach einem der Ansprüche 1 bis 3,
**dadurch gekennzeichnet, dass** die beiden Haltearme (1, 2) in einer Verbindungsstelle (9) mit ihren freien Armenden (3, 4) gegeneinander schwenkbar verbunden sind und die Spreizeinrichtung (20; 21) an wenigstens einem der beiden Haltearme (1, 2) angreift.

5. Vorrichtung nach einem der Ansprüche 1 bis 4,
**dadurch gekennzeichnet, dass** die Vorspannkraft in der Verbindungsstelle gebildet ist.

6. Vorrichtung nach einem der Ansprüche 1 bis 5,
**dadurch gekennzeichnet, dass** die Verbindungsstelle durch einen Steg (12) gebildet ist.

7. Vorrichtung nach einem der Ansprüche 1 bis 6,
**dadurch gekennzeichnet, dass** wenigstens einer der beiden Haltearme (1, 2) als einseitiger oder zweiseitiger Hebel ausgebildet ist, an welchem die Spreizeinrichtung (20; 21) angreift.

8. Vorrichtung nach Anspruch 7,
**dadurch gekennzeichnet, dass** der wenigstens eine Haltearm als geradliniger zweiseitiger Hebel ausgebildet ist.

9. Vorrichtung nach Anspruch 8,
**dadurch gekennzeichnet, dass** der wenigstens eine Haltearm als ein- oder zweiseitiger Winkelhebel ausgebildet ist.

10. Vorrichtung nach einem der Ansprüche 1 bis 9,
**dadurch gekennzeichnet, dass** die Spreizeinrichtung (20; 21; 25) als ortsfester Anschlag im Gehäuseinnern ausgebildet ist.

11. Vorrichtung nach einem der Ansprüche 1 bis 9,
**dadurch gekennzeichnet, dass** einer der beiden Haltearmen mit dem Behälterverschluss (14) verbunden ist.

12. Vorrichtung nach einem der Ansprüche 1 bis 11,
**dadurch gekennzeichnet, dass** die Vorspannkraft durch ein auf die Haltearme (1, 2) einwirkendes federelastisches Element (24) gebildet ist.

13. Vorrichtung nach einem der Ansprüche 1 bis 12,
**dadurch gekennzeichnet, dass** an wenigstens einem Haltearm (1, 2) ein Impulsgeber (27) vorgesehen ist, durch welchen infolge der Vorspannung ein mechanischer Impuls auf die Linse (6) zum Falten in einer bestimmten Richtung ausgeübt wird.

## Claims

1. A device for holding a foldable intraocular lens comprising two mutually pivotable holding arms, between which a biasing force acts and which, arranged in a container, hold the lens in the unfolded state in the proximity of the free ends or at the free ends, wherein upon removal from the container the lens is folded by the holding arms moving towards each other, **characterised in that** the free ends (3, 4) of the holding arms (1, 2) which are biased towards each other by the biasing force, when arranged in the container (5), are spread against the biasing force to hold the lens (6) in the unfolded state and the biasing force is of such a magnitude that upon removal of the holding arms from the container (5) the lens (6) held between the holding arms (1, 2) is folded.

2. A device according to claim 1 **characterised in that** provided in the container (5) is a spreading device (20; 21) by which the holding arms (1, 2) are spread apart against the biasing force to hold the lens in the unfolded state.

3. A device according to claim 1 or claim 2 **characterised in that** the two holding arms (1, 2) are formed from one piece.

4. A device according to one of claims 1 to 3 **characterised in that** the two holding arms (1, 2) are connected pivotably relative to each other with their free arm ends (3, 4) at a connecting location (9) and the spreading device (20; 21) engages at least one of the two holding arms (1, 2).

5. A device according to one of claims 1 to 4 **characterised in that** the biasing force is formed in the connecting location.

6. A device according to one of claims 1 to 5 **characterised in that** the connecting location is formed by a limb (12).

7. A device according to one of claims 1 to 6 **characterised in that** at least one of the two holding arms (1, 2) is in the form of a single-sided or double-sided lever which is engaged by the spreading device (20; 21).

8. A device according to claim 7 **characterised in that** the at least one holding arm is in the form of a straight double-sided lever.

9. A device according to claim 8 **characterised in that** the at least one holding arm is in the form of a single-sided or double-sided angular lever.

10. A device according to one of claims 1 to 9 **characterised in that** the spreading device (20; 21; 25) is in the form of a stationary abutment in the interior of the housing.

11. A device according to one of claims 1 to 9 **characterised in that** one of the two holding arms is connected to the container closure (14).

12. A device according to one of claims 1 to 11 **characterised in that** the biasing force is formed by a resilient element (24) acting on the holding arms (1, 2).

13. A device according to one of claims 1 to 12 **characterised in that** provided at at least one holding arm (1, 2) is an impulse-producing means (27) by which as a consequence of the biasing effect a mechanical impulse is exerted on the lens (7) for folding in a given direction.

## Revendications

1. Dispositif de maintien d'une lentille intraoculaire pliable, comprenant deux bras de maintien pouvant pivoter l'un par rapport à l'autre, entre lesquels agit une précontrainte et qui, disposés dans un récipient, maintiennent la lentille à l'état déplié, à proximité des extrémités libres ou sur les extrémités libres, la lentille étant pliée sous l'effet du rapprochement des bras de maintien l'un de l'autre lors de l'enlèvement du récipient,
**caractérisé en ce que** les extrémités libres (3, 4) des bras de maintien (1, 2), lesquels sont soumis à la précontrainte les rapprochant l'un de l'autre, sont écartées à l'encontre de la précontrainte lors de la disposition dans le récipient (5) pour maintenir la lentille (6) à l'état dépliée, et **en ce que** la force de précontrainte est telle que, lorsque les bras de maintien sont enlevés du récipient (5), la lentille (6) maintenue entre les bras de maintien (1, 2) est pliée.

2. Dispositif selon la revendication 1,
**caractérisé en ce que** dans le récipient (5) est prévu un dispositif d'écartement (20 ; 21), au moyen duquel les bras de maintien (1, 2) sont écartés l'un de l'autre à l'encontre de la précontrainte pour maintenir la lentille à l'état dépliée.

3. Dispositif selon la revendication 1 ou 2,
**caractérisé en ce que** les deux bras de maintien (1, 2) sont formés d'une seule pièce.

4. Dispositif selon l'une des revendications 1 à 3,
**caractérisé en ce que** les deux bras de maintien (1, 2) sont reliés en un point de liaison (9) de façon à pouvoir pivoter l'un vers l'autre par leurs extrémités de bras (3, 4) libres et **en ce que** le dispositif d'écartement (20 ; 31) agit sur au moins l'un des deux bras de maintien (1, 2).

5. Dispositif selon l'une des revendications 1 à 4,
**caractérisé en ce que** la force de précontrainte est appliquée au point de liaison.

6. Dispositif selon l'une des revendications 1 à 5,
**caractérisé en ce que** le point de liaison est constitué par une entretoise (12).

7. Dispositif selon l'une des revendications 1 à 6,
**caractérisé en ce qu'**au moins l'un des deux bras de maintien (1, 2) est réalisé sous la forme d'un levier unilatéral ou bilatéral, sur lequel agit le dispositif d'écartement (20 ; 21).

8. Dispositif selon la revendication 7,
**caractérisé en ce que** le au moins un bras de maintien est réalisé sous la forme d'un levier bilatéral rectiligne.

9. Dispositif selon la revendication 8,
**caractérisé en ce que** le au moins un bras de maintien est réalisé sous la forme d'un levier coudé unilatéral ou bilatéral.

10. Dispositif selon l'une des revendications 1 à 9,
**caractérisé en ce que** le dispositif d'écartement (20 ; 21 ; 25) est réalisé sous la forme d'une butée fixe à l'intérieur du boîtier.

11. Dispositif selon l'une des revendications 1 à 9,
**caractérisé en ce que** l'un des deux bras de maintien est relié à la fermeture (14) du récipient.

12. Dispositif selon l'une des revendications 1 à 11,
**caractérisé en ce que** la force de précontrainte est appliquée par un élément (24) à élasticité de ressort, agissant sur les bras de maintien (1, 2).

13. Dispositif selon l'une des revendications 1 à 12,
**caractérisé en ce que** sur au moins un bras de maintien (1, 2) est prévu un générateur d'impulsions (27) au moyen duquel, sous l'effet de la précontrainte, une quantité de mouvement mécanique est exercée sur la lentille (6) pour le pliage, dans une direction déterminée.
